# FASCICULE DE BREVET EUROPEEN

(11) **EP 0 854 858 B1**
(45) Date de publication et mention de la délivrance du brevet: **13.06.2001**
(21) Numéro de dépôt: 96932661.0
(22) Date de dépôt: 27.09.1996
(51) Int. Cl.: C07C 253/10, C07C 255/04, B01J 31/24, C07F 9/50

(54) **PROCEDE D'HYDROCYANATION DE COMPOSES ORGANIQUES A INSATURATION ETHYLENIQUE**
VERFAHREN ZUR HYDROCYANIERUNG VON ORGANISCHEN, ETHYLENISCH UNGESÄTTIGTEN VERBINDUNGEN
ETHYLENICALLY UNSATURATED ORGANIC COMPOUND HYDROCYANATION METHOD

(30) Priorité: 29.09.1995 FR 9511689
(43) Date de publication de la demande: 29.07.1998
(73) Titulaire: RHODIA POLYAMIDE INTERMEDIATES, 69190 Saint-Fons (FR)
(72) Inventeur: HUSER, Marc, F-69100 Villeurbanne (FR); PERRON, Robert, F-69390 Charly (FR)
(74) Mandataire: Esson, Jean-Pierre
(86) Numéro de dépôt international: FR9601509
(87) Numéro de publication internationale: WO9712857

(56) Documents cités:
- EP-A- 0 133 127
- EP-A- 0 647 619
- EP-A- 0 650 959
- WO-A-95/22405
- WO-A-95/30680
- FR-A- 2 033 107
- FR-A- 2 069 411
- FR-A- 2 338 253

## Description

La présente invention concerne un procédé d'hydrocyanation de composés organiques à insaturation éthylénique en nitriles, notamment l'hydrocyanation des dioléfines ou des oléfines substituées telles que des alcène-nitriles.

Le brevet français n° 1 599 761 décrit un procédé de préparation de nitriles par addition d'acide cyanhydrique sur des composés organiques ayant au moins une double liaison éthylénique, en présence d'un catalyseur au nickel et d'un phosphite de triaryle. Cette réaction peut être conduite en présence ou non d'un solvant.

Lorsqu'un solvant est utilisé dans ce procédé de l'art antérieur, il s'agit de préférence d'un hydrocarbure, tel que le benzène ou les xylènes ou d'un nitrile tel que l'acétonitrile.

Le catalyseur mis en oeuvre est un complexe organique de nickel, contenant des ligands tels que les phosphines, les arsines, les stilbines, les phosphites, les arsénites ou les antimonites.

La présence d'un promoteur pour activer le catalyseur, tel qu'un composé du bore ou un sel métallique, généralement un acide de Lewis, est également préconisée dans ledit brevet.

Dans ce procédé, le milieu est entièrement organique et l'un de ses inconvénients majeurs réside dans la difficulté de séparer, en fin de réaction, les produits de l'hydrocyanation, de la solution catalytique contenant plusieurs constituants (complexe du nickel, phosphite de triaryle, promoteur), en vue notamment du recyclage de cette dernière dans une nouvelle réaction d'hydrocyanation. Une telle séparation est délicate, très complexe et imparfaite et on constate une perte substantielle de catalyseur, ainsi que la présence dudit catalyseur dans les produits d'hydrocyanation. Cette perte de catalyseur métallique, généralement à base de nickel, pose des problèmes économiques, mais soulève de manière encore plus pressante des questions sur le devenir de tels métaux, car le rejet d'effluents ou le stockage de déchets sont de moins en moins acceptables pour l'environnement.

Le brevet FR-A-2 338 253 a proposé de réaliser l'hydrocyanation des composés ayant au moins une insaturation éthylénique, en présence d'une solution aqueuse d'un composé d'un métal de transition, notamment le nickel, le palladium ou le fer, et d'une phosphine sulfonée.

Les phosphines sulfonées décrites dans ce brevet sont des triarylphosphines sulfonées et plus particulièrement des triphénylphosphines sulfonées.

Ce procédé permet une bonne hydrocyanation, notamment du butadiène et des pentène-nitriles, une séparation aisée de la solution catalytique par simple décantation et par conséquent évite au maximum le rejet d'effluents ou de déchets contenant les métaux servant de catalyseur.

Les résultats obtenus lors de la réaction d'hydrocyanation sont relativement bons avec les différents substrats et notamment avec les oléfines fonctionnalisées comme les pentène-nitriles. Il s'avère cependant que la durée de vie du catalyseur pourrait être améliorée afin de permettre une exploitation industrielle de ce type de procédé.

Le brevet EP-A-0 650 959 décrit un procédé d'hydrocyanation de nitriles insaturés en dinitriles en présence des mêmes phosphines sulfonées que dans le brevet précédent et d'un acide de Lewis.

Le brevet EP-A-0 647 619 décrit l'isomérisation du méthyl-2 butène-3 nitrile en pentene-nitrile linéaire en utilisant le système catalytique décrit dans FR-A-2 338 253.

Le brevet EP-A-0 133 127 décrit de nouvelles phosphines chirales sulfonées. Ces phosphines chirales sulfonées sont utilisées sous forme de complexes du rhodium en catalyse asymétrique, essentiellement en hydrogénation asymétrique d'acides acryliques substitués. Ces possiblités de catalyse asymétrique et plus particulièrement d'hydrogénation asymétrique ne présentent pas d'intérêt pour l'hydrocyanation de composés éthyléniques.

La demande WO-A-95/22405 décrit des catalyseurs BINAP (composés 2,2'-bis(diphénylphosphino)-1,1'-binaphtyl sulfonés) hydrosolubles utilisables pour la synthèse asymétrique de composés optiquement actifs

La présente invention concerne un procédé susceptible de fournir une solution industrielle performante à cette très importante réaction d'hydrocyanation, qui conduit lorsqu'elle s'applique par exemple au butadiène, puis aux pentène-nitriles à l'adiponitrile, l'un des composés de base nécessaires à la fabrication du polyamide-6,6.

Plus précisément, elle consiste en un procédé d'hydrocyanation de composés organiques comportant au moins une double liaison éthylénique par réaction avec le cyanure d'hydrogène, en présence d'une solution aqueuse d'un catalyseur comprenant au moins un composé d'un métal de transition et au moins une phosphine hydrosoluble, caractérisé en ce que ladite phosphine hydrosoluble est une phosphine monodentate ou bidentate répondant à la formule générale (I) : 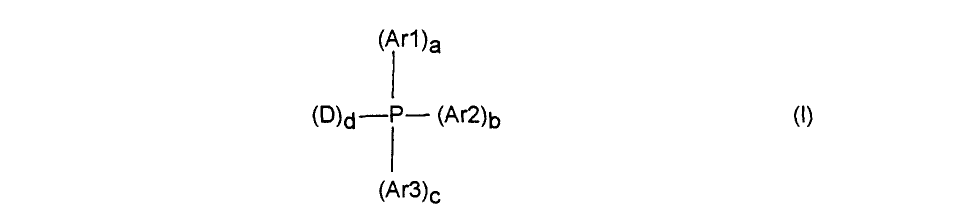 dans laquelle :
- Ar1 et Ar2, identiques ou différents, représentent des groupes aryle ou aryle comportant un ou plusieurs substituants tels que :
   - radical alkyle ou alcoxy ayant 1 à 4 atomes de carbone,
   - atome d'halogène,
   - groupe hydrophile comme :
      - COOM, -SO₃M, -PO₃M, M représentant un reste cationique minéral ou organique choisi parmi le proton, les cations dérivés des métaux alcalins ou alcalino-terreux, les cations ammonium -N(R)₄ dans la formule desquels les symboles R, identiques ou différents, représentent un atome d'hydrogène ou un radical alkyle ayant de 1 à 4 atomes de carbone, les autres cations dérivés de métaux dont les sels des acides arylcarboxyliques, des acides arylsulfoniques ou des acides arylphosphoniques sont solubles dans l'eau,
      - N(R)3 dans la formule duquel les symboles R, identiques ou différents, représentent un atome d'hydrogène ou un radical alkyle ayant de 1 à 4 atomes de carbone,
      - OH
- Ar3 représente un groupe aryle comportant un ou plusieurs substituants tels que :
   - radical alkyle ou alcoxy ayant 1 à 4 atomes de carbone,
   - atome d'halogène,
   - groupe hydrophile comme :
      - COOM, -PO3M, M représentant un reste cationique minéral ou organique choisi parmi le proton, les cations dérivés des métaux alcalins ou alcalino-terreux,
      les cations ammonium -N(R)4 dans la formule desquels les symboles R, identiques ou différents, représentent un atome d'hydrogène ou un radical alkyle ayant de 1 à 4 atomes de carbone, les autres cations dérivés de métaux dont les sels des acides arylcarboxyliques ou des acides arylphosphoniques sont solubles dans l'eau,
   l'un au moins desdits substituants de Ar3 étant un groupe hydrophile tel que défini précédemment,
   - a représente 0 ou 1,
   - b représente 0 ou 1,
   - c représente un nombre entier de 0 à 3,
   - D représente un groupe alkyle, un groupe cycloalkyle, un groupe alkyle ou cycloalkyle comportant un ou plusieurs substituants tels que :
      - radical alcoxy ayant 1 à 4 atomes de carbone,
      - atome d'halogène,
      - groupe hydrophile comme :
         - COOM, -SO3M, -PO3M, M représentant un reste cationique minéral ou organique choisi parmi le proton, les cations dérivés des métaux alcalins ou alcalino-terreux, les cations ammonium -N(R)4 dans la formule desquels les symboles R, identiques ou différents, représentent un atome d'hydrogène ou un radical alkyle ayant de 1 à 4 atomes de carbone, les autres cations dérivés de métaux dont les sels des acides arylcarboxyliques, des acides arylsulfoniques ou des acides phosphoniques sont solubles dans l'eau,
      - N(R)3 dans la formule duquel les symboles R, identiques ou différents, représentent un atome d'hydrogène ou un radical alkyle ayant de 1 à 4 atomes de carbone,
      - OH

   - d représente un nombre entier de 0 à 3,
   - la somme (a+b+c+d) est égale à 3
      ou à la formule générale (II) : 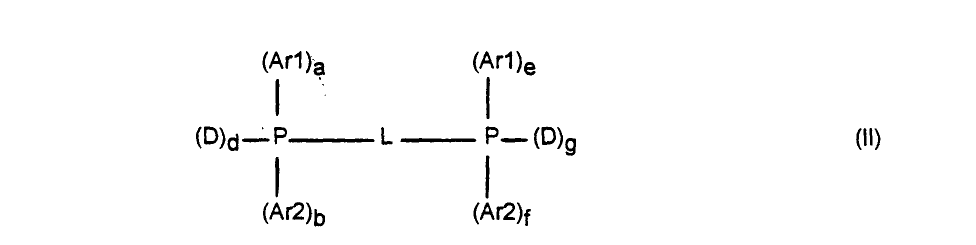 dans laquelle :
      - Ar1, Ar2 et D ont les significations indiquées précédemment pour la formule (I),
      - a, b, e et f représentent chacun 0 ou 1,
      - d et g représentent chacun un nombre entier de 0 à 2,
      - la somme (a+b+d) est égale à 2,
      - la somme (e+f+g) est égale à 2,
      - L représente un lien valentiel simple ou un radical hydrocarboné divalent tel qu'un radical alkylène, un radical cycloalkylène, un radical arylène, un radical dérivant d'un hétérocycle comportant un ou deux atomes d'oxygène, d'azote ou de soufre dans le cycle, ces différents radicaux cycliques étant liés directement à l'un des atomes de phosphore ou aux deux atomes de phosphore ou étant lié à l'un des atomes de phosphore ou aux deux par l'intermédiaire d'un radical alkylène linéaire ou ramifié ayant de 1 à 4 atomes de carbone, le ou les cycles éventuellement parties du radical divalent L pouvant comporter un ou plusieurs substituants tels que groupe alkyle ayant 1 à 4 atomes de carbone.

On peut citer, comme exemples de métaux dont les sels des acides arylcarboxyliques, des acides arylsulfoniques ou des acides arylphosphoniques sont solubles dans l'eau, le plomb, le zinc et l'étain.

Par l'expression soluble dans l'eau, on entend dans le présent texte un composé soluble à au moins 0,01 g par litre d'eau.

Les phosphines hydrosolubles préférées sont les phosphines de formule (I) ou de formule (II) dans lesquelles Ar1 et Ar2 sont des groupes phényle ou des groupes phényle comportant un ou deux substituants tels que définis précédemment, Ar3 est un groupe phényle comportant un ou deux substituants tels que définis précédemment, D est groupe alkyle ayant de 1 à 6 atomes de carbone, un groupe cycloalkyle ayant 5 à 8 atomes de carbone, un groupe alkyle ayant de 1 à 6 atomes de carbone ou cycloalkyle ayant 5 à 8 atomes de carbone comportant un ou plusieurs substituants tels que définis précédemment, L est un lien valentiel simple, un radical alkylène ayant de 1 à 6 atomes de carbone, un radical cycloalkylène monocyclique ou bicyclique ayant de 4 à 12 atomes de carbone, un radical phénylène, un radical diphénylène, un radical naphtylène, un radical dinaphtylène, un radical dérivant d'un hétérocycle comportant un ou deux atomes d'oxygène, d'azote ou de soufre dans le cycle, ces différents radicaux cycliques étant liés directement à l'un des atomes de phosphore ou aux deux atomes de phosphore ou étant lié à l'un des atomes de phosphore ou aux deux par l'intermédiaire d'un radical alkylène linéaire ou ramifié ayant de 1 à 4 atomes de carbone, le ou les cycles éventuellement parties du radical divalent L pouvant comporter un ou plusieurs substituants tels que groupe alkyle ayant 1 à 4 atomes de carbone.

Les phosphines hydrosolubles préférées sont les phosphines de formule (I) ou de formule (II) dans lesquelles:
- le ou les substituants de Ar1 et Ar2, identiques ou différents, représentent des groupes tels que :
   - radical alkyle ou alcoxy ayant 1 à 2 atomes de carbone,
   - atome de chlore,
   - groupe hydrophile comme :
      - COOM, -SO₃M, -PO₃M, M représentant un reste cationique minéral ou organique choisi parmi le proton, les cations dérivés du sodium, du potassium, du calcium ou du baryum, les cations ammonium, tétraméthylammonium, tétraéthylammonium, tétrapropylammonium, tétrabutylammonium, les cations dérivés du zinc, du plomb ou de l'étain,
      - N(R)₃ dans la formule duquel les symboles R, identiques ou différents, représentent un atome d'hydrogène ou un radical alkyle ayant de 1 à 4 atomes de carbone,
      - OH
- le ou les substituants de Ar3, identiques ou différents, représentent des groupes tels que :
   - radical alkyle ou alcoxy ayant 1 à 2 atomes de carbone,
   - atome de chlore,
   - groupe hydrophile comme :
      - COOM, -PO₃M, M représentant un reste cationique minéral ou organique choisi parmi le proton, les cations dérivés du sodium, du potassium, du calcium ou du baryum, les cations ammonium, tétraméthylammonium, tétraéthylammonium, tétrapropylammonium, tétrabutylammonium, les cations dérivés du zinc, du plomb ou de l'étain, globalement deux au moins desdits substituants de Ar1, Ar2, Ar3 et D pour les phosphines de formule (I) et de Ar1, Ar2 et D pour les phosphines de formule (II) étant un groupe hydrophile tel que défini précédemment.

A titre d'exemples non limitatifs de phosphines de formule générale (I), on peut citer notamment la tris(hydroxyméthyl) phosphine, la tris(2-hydroxyéthyl) phosphine, la tris(3-hydroxypropyl) phosphine, la tris(2-carboxyméthyl) phosphine, le sel de sodium de la tris(3-carboxyphényl) phosphine, la tris(3-carboxyéthyl) phosphine, l'iodure de la tris(4-triméthylammoniumphényl) phosphine, le sel de sodium de la tris(2-phosphonatoéthyl) phosphine, la bis(2-carboxyéthyl) phényl phosphine, le sel de sodium de la tris(paraphosphophényl) phosphine, le sel de sodium de la bis(métasulfophényl) paracarboxyphényl phosphine, le sel de sodium de la bis(métasulfophényl) sulfo-2 éthyl phosphine.

A titre d'exemples non limitatifs de phosphines de formule générale (II), on peut citer notamment le sel de sodium du 2,2'-bis[di(sulfonatophényl)phosphino]-1,1'-binaphtyl, le sel de sodium du 1,2-bis[di(sulfonatophényl)phosphinométhyl] cyclobutane (CBDTS), le 1,2-bis(dihydroxyméthyl-phosphino) éthane,
le 1,3-bis(dihydroxyméthylphosphino) propane, le sel de sodium du
2,2'-bis[di(sulfonatophényl)phosphinométhyl]-1,1'-binaphtyl.

Certaines des phosphines hydrosolubles de formule (I) ou (II) sont disponibles dans le commerce.

Pour la préparation des autres, on peut se référer aux procédés généraux ou particuliers de synthèse des phosphines décrits dans les ouvrages généraux comme HOUBEN-WEYL, Method der organischen Chemie, organische Phosphor Verbindungen, teil 1 (1963).

Enfin, pour la préparation des dérivés hydrosolubles non décrits, on peut à partir des phosphines ne comportant pas de substituants hydrosolubles définis précédemment, procéder à l'introduction d'un ou plusieurs de ces substituants hydrophiles. Ainsi les groupements sulfonates par exemple peuvent être introduits par la réaction de SO₃ dans l'acide sulfurique. Les groupes carboxylates, phosphonates, ammonium quaternaires peuvent de même être introduits en appliquant les méthodes chimiques connues pour ce type de synthèse.

On utilise de préférence comme composés des métaux de transition des composés du nickel, du palladium et du fer. On utilise des composés solubles dans l'eau ou capables de passer en solution dans les conditions de la réaction. Le reste lié au métal n'est pas critique, dès l'instant qu'il satisfait à ces conditions.

Parmi les composés précités, les composés les plus préférés sont ceux du nickel, on peut citer à titre d'exemples non limitatifs :
- les composés dans lesquel le nickel est au degré d'oxydation zéro comme le tétracyanonickelate de potassium K₄ [Ni(CN)₄], le bis (acrylonitrile) nickel zéro, le bis (cyclooctadiène-1,5)₂ nickel et les dérivés contenant des ligands du groupe Va comme le tétrakis (triphényl-phosphine) nickel zéro (dans ce dernier cas le composé peut être dissous dans un solvant non miscible à l'eau comme le toluène, puis une solution aqueuse de phosphine sulfonée extrait une partie du nickel en développant une coloration rouge dans la solution aqueuse qui décante);
- les composés du nickel comme les carboxylates (notamment l'acétate), carbonate, bicarbonate, borate, bromure, chlorure, citrate, thiocyanate, cyanure, formiate, hydroxyde, hydrophosphite, phosphite, phosphate et dérivés, iodure, nitrate, sulfate, sulfite, aryl- et alkyl-sulfonates.

Il n'est pas nécessaire que le composé du nickel soit lui même soluble dans l'eau. Par exemple, le cyanure de nickel peu soluble dans l'eau se dissout très bien dans une solution aqueuse de phosphine.

Quand le composé du nickel utilisé correspond à un état d'oxydation du nickel supérieur à 0, on ajoute au milieu réactionnel un réducteur du nickel réagissant préférentiellement avec le nickel dans les conditions de la réaction. Ce réducteur peut être organique ou minéral. On peut citer comme exemples non limitatifs le BH₄Na, la poudre de Zn, le magnésium, le BH₄K et les borohydrures de préférence solubles dans l'eau.

Ce réducteur est ajouté en quantité telle que le nombre d'équivalents d'oxydoréduction est compris entre 1 et 10. Des valeurs inférieures à 1 et supérieures à 10 ne sont toutefois pas exclues.

Lorsque le composé du nickel utilisé correspond à l'état d'oxydation 0 du nickel, on peut également ajouter un réducteur du type de ceux précités, mais cet ajout n'est pas impératif.

Lorsqu'on utilise un composé du fer, les mêmes réducteurs conviennent.

Dans le cas du palladium, les réducteurs peuvent être, en outre, des éléments du milieu réactionnel (phosphine, solvant, oléfine).

Les composés organiques comportant au moins une double liaison éthylénique plus particulièrement mis en oeuvre dans le présent procédé sont les dioléfines comme le butadiène, l'isoprène, l'hexadiène-1,5, le cyclooctadiène-1,5, les nitriles aliphatiques à insaturation éthylénique, particulièrement les pentène-nitriles linéaires comme le pentène-3 nitrile, le pentène-4 nitrile, les monooléfines comme le styrène, le méthyl-styrène, le vinyl-naphtalène, le cyclohexène, le méthyl-cyclohexène ainsi que les mélanges de plusieurs de ces composés.

Les pentène-nitriles notamment peuvent contenir des quantités, généralement minoritaires, d'autres composés, comme le méthyl-2 butène-3 nitrile, le méthyl-2 butène-2 nitrile, le pentène-2 nitrile, le valéronitrile, l'adiponitrile, le méthyl-2 glutaronitrile, l'éthyl-2 succinonitrile ou le butadiène, provenant par exemple de la réaction antérieure d'hydrocyanation du butadiène.

Lors de l'hydrocyanation du butadiène, il se forme avec les pentène-nitriles linéaires des quantités non négligeables de méthyl-2 butène-3 nitrile et de méthyl-2 butène-2 nitrile.

La solution catalytique utilisée pour l'hydrocyanation selon le procédé de l'invention peut être préparée avant son introduction dans la zone de réaction, par exemple par addition à la solution aqueuse de la phosphine hydrosoluble de formule (I) ou (II), de la quantité appropriée de composé du métal de transition choisi et éventuellement du réducteur. Il est également possible de préparer la solution catalytique "in situ" par simple mélange de ces divers constituants.

La quantité de composé du nickel ou d'un autre métal de transition utilisée est choisie de telle sorte qu'il y ait par litre de solution réactionnelle entre 10⁻⁴ et 1, et de préférence entre 0,005 et 0,5 mole de nickel ou de l'autre métal de transition mis en oeuvre.

La quantité de phosphine hydrosoluble de formule (I) ou (II) utilisée pour préparer la solution réactionnelle est choisie de telle sorte que le nombre de moles de ce composé rapporté à 1 mole de métal de transition soit de 0,5 à 2000 et de préférence de 2 à 300.

Bien que la réaction soit conduite généralement sans tiers-solvant, il peut être avantageux de rajouter un solvant organique inerte, non miscible à l'eau, qui pourra être celui de l'extraction ultérieure.

A titre d'exemples de tels solvants, on peut citer les hydrocarbures aromatiques, aliphatiques ou cycloaliphatiques qui maintiennent à l'état biphasique le milieu réactionnel.

Ainsi dès la fin de la réaction, il est très aisé de séparer, d'une part, une phase aqueuse contenant la phosphine ou les phosphines hydrosolubles de formule (I) ou (II) et le composé de métal de transition et d'autre part, une phase organique constituée des réactifs engagés dans la réaction des produits de la réaction et le cas échéant du solvant organique non miscible à l'eau.

La réaction d'hydrocyanation est généralement réalisée à une température de 10°C à 200°C et de préférence de 30°C à 120°C.

Le procédé de l'invention peut être mis en oeuvre de manière continue ou discontinue.

Le cyanure d'hydrogène mis en oeuvre peut être préparé à partir des cyanures métalliques, notamment le cyanure de sodium, ou des cyanhydrines.

Le cyanure d'hydrogène est introduit dans le réacteur sous forme gazeuse ou sous forme liquide. Il peut également être préalablement dissous dans un solvant organique.

Dans le cadre d'une mise en oeuvre discontinue, on peut en pratique charger dans un réacteur, préalablement purgé à l'aide d'un gaz inerte (tel qu'azote, argon), soit une solution aqueuse contenant la totalité ou une partie des divers constituants tels que la phosphine hydrosoluble, le composé de métal de transition, les éventuels réducteur et solvant, soit séparément lesdits constituants. Généralement le réacteur est alors porté à la température choisie, puis le pentène-nitrile est introduit. Le cyanure d'hydrogène est alors lui-même introduit, de préférence de manière continue et régulière.

Lorsque la réaction (dont on peut suivre l'évolution par dosage de prélèvements) est terminée, le mélange réactionnel est soutiré après refroidissement et les produits de la réaction sont isolés par décantation, éventuellement suivie d'une extraction de la couche aqueuse à l'aide d'un solvant approprié, tel que par exemple les solvants non miscibles à l'eau cités précédemment.

La solution catalytique aqueuse peut alors être recyclée dans une nouvelle réaction d'hydrocyanation de composés organiques comportant au moins une double liaison éthylénique.

Dans le cadre d'une mise en oeuvre du procédé en continu, seule la phase organique peut être soutirée, tandis que la phase catalytique aqueuse demeure dans le réacteur.

Cette réaction en milieu biphasique rend extrêmement simple la mise en oeuvre d'un procédé industriel. La séparation aisée de la totalité du catalyseur d'une part et des produits de la réaction d'autre part, rend inutiles un grand nombres d'opérations telles que la distillation des réactifs et des produits de la réaction ou l'extraction liquide/liquide à l'aide d'un solvant approprié.

Un perfectionnement au procédé d'hydrocyanation de composés à insaturation éthylénique selon la présente invention concerne l'hydrocyanation desdits composés à insaturation éthylénique, par réaction avec le cyanure d'hydrogène, caractérisée en ce que l'on opère en présence d'une solution aqueuse d'un catalyseur comprenant au moins un composé d'un métal de transition, au moins une phosphine hydrosoluble de formule (I) ou (II) et un cocatalyseur consistant en au moins un acide de Lewis.

Les composés à insaturation éthylénique qui peuvent être mis en oeuvre dans ce perfectionnement sont de manière générale ceux qui ont été cités pour le procédé de base. Cependant il est plus particulièrement avantageux de l'appliquer aux nitriles aliphatiques à insaturation éthylénique, notamment aux pentène-nitriles linéaires comme le pentène-3 nitrile, le pentène-4 nitrile et leurs mélanges

Ces pentène-nitriles peuvent contenir des quantités, généralement minoritaires, d'autres composés, comme le méthyl-2 butène-3 nitrile, le méthyl-2 butène-2 nitrile, le pentène-2 nitrile, le valéronitrile, l'adiponitrile, le méthyl-2 glutaronitrile, l'éthyl-2 succinonitrile ou le butadiène, provenant de la réaction antérieure d'hydrocyanation du butadiène et/ou de l'isomérisation du méthyl-2 butène-3 nitrile en pentène-nitriles.

Les phosphines hydrosolubles, les composés de métal de transition, les conditions opératoires, la composition du milieu réactionnel sont les mêmes que pour le procédé général d'hydrocyanation selon l'invention qui a été décrit précédemment, mais la réaction est conduite en outre en présence d'un acide de Lewis.

L'acide de Lewis utilisé comme cocatalyseur permet notamment, dans le cas de l'hydrocyanation des nitriles aliphatiques à insaturation éthylénique, d'améliorer la linéarité des dinitriles obtenus, c'est-à-dire le pourcentage de dinitrile linéaire par rapport à la totalité des dinitriles formés, et/ou d'augmenter la durée de vie du catalyseur.

Par acide de Lewis, on entend dans le présent texte, selon la définition usuelle, des composés accepteurs de doublets électroniques.

On peut mettre en oeuvre notamment les acides de Lewis cités dans l'ouvrage édité par G.A. OLAH "Friedel-Crafts and related Reactions", tome I, pages 191 à 197 (1963).

Les acides de Lewis qui peuvent être mis en oeuvre comme cocatalyseurs dans le présent procédé sont choisis parmi les composés des éléments des groupes Ib, llb, Illa, Illb, IVa, IVb, Va, Vb, Vlb, Vllb et VIII de la Classification périodique des éléments, dans la mesure où lesdits composés sont au moins partiellement solubles et stables dans l'eau. Ces composés sont le plus souvent des sels, notamment des halogénures, de préférence chlorures et bromures, des sulfates, des carboxylates et des phosphates.

A titre d'exemples non limitatifs de tels acides de Lewis, on peut citer le chlorure de zinc, le bromure de zinc, l'iodure de zinc, le chlorure de manganèse, le bromure de manganèse, le chlorure de cadmium, le bromure de cadmium, le chlorure stanneux, le bromure stanneux, le sulfate stanneux, le tartrate stanneux, les chlorures ou bromures des éléments des terres rares comme le lanthane, le cérium, le praséodyme, le néodyme, le samarium, l'europium, le gadolinium, le terbium, le dysprosium, l'holmium, l'erbium, le thulium, l'ytterbium et le lutétium, le chlorure de cobalt, le chlorure ferreux, le chlorure d'yttrium.

On peut bien entendu mettre en oeuvre des mélanges de plusieurs acides de Lewis.

Il est également intéressant le cas échéant de stabiliser l'acide de Lewis en solution aqueuse par l'adjonction d'un chlorure de métal alcalin comme le chlorure de lithium ou le chlorure de sodium notamment. Le rapport molaire chlorure de lithium ou de sodium/acide de Lewis varie de manière très large, par exemple de 0 à 100, le rapport particulier pouvant être ajusté selon la stabilité dans l'eau de l'acide de Lewis.

Parmi les acides de Lewis, on préfére tout particulièrement le chlorure de zinc, le bromure de zinc, le chlorure stanneux, le bromure stanneux, le chlorure stanneux stabilisé par le chlorure de lithium, le chlorure stanneux stabilisé par le chlorure de sodium, les mélanges chlorure de zinc/chlorure stanneux.

Le cocatalyseur acide de Lewis mis en oeuvre représente généralement de 0,01 à 50 moles par mole de composé de métal de transition, plus particulièrement de composé du nickel, et de préférence de 1 à 10 mole par mole.

Comme pour la mise en oeuvre du procédé de base de l'invention, la solution catalytique utilisée pour l'hydrocyanation en présence d'acide de Lewis peut être préparée avant son introduction dans la zone de réaction, par exemple par addition à la solution aqueuse de la phosphine hydrosoluble de formule (I) ou (II), de la quantité appropriée de composé du métal de transition choisi, de l'acide de Lewis et éventuellement du réducteur. Il est également possible de préparer la solution catalytique "in situ" par simple mélange de ces divers constituants.

La séparation du catalyseur en phase aqueue est aisée par une simple décantation, comme cela a été indiqué précédemment pour le procédé de base. Cette solution aqueuse de catalyseur peut ainsi être recyclée dans une nouvelle réaction d'hydrocyanation. Dans le cas, très important sur le plan économique, de l'hydrocyanation des pentène-nitriles, on peut recycler cette solution de catalyseur, soit dans une nouvelle hydrocyanation de pentène-nitriles, soit plus généralement dans l'hydrocyanation du butadiène conduisant aux pentène-nitriles qui sont ensuite hydrocyanés avec le même catalyseur.

II est également possible dans les conditions du procédé d'hydrocyanation de la présente invention, et notamment en opérant en présence du catalyseur décrit précédemment comportant au moins une phosphine hydrosoluble de formule (I) ou (II) et au moins un composé d'un métal de transition, de réaliser, en absence de cyanure d'hydrogène, l'isomérisation du méthyl-2 butène-3 nitrile en pentène-nitriles.

Le méthyl-2 butène-3 nitrile soumis à l'isomérisation selon l'invention peut être mis en oeuvre seul ou en mélange avec d'autres composés.

Ainsi on peut engager du méthyl-2 butène-3 nitrile en mélange avec du méthyl-2 butène-2 nitrile, du pentène-4 nitrile, du pentène-3 nitrile, du pentène-2 nitrile, du butadiène, de l'adiponitrile, du méthyl-2 glutaroronitrile, de l'éthyl-2 succinonitrile ou du valéronitrile.

Ainsi, il est particulièrement intéressant de traiter le mélange réactionnel provenant de l'hydrocyanation du butadiène par HCN en présence d'une solution aqueuse d'au moins une phosphine hydrosoluble de formule (I) ou (II) et d'au moins un composé d'un métal de transition, plus préférentiellement d'un composé du nickel au degré d'oxydation 0, tel que défini précédemment.

Dans le cadre de cette variante préférée, le système catalytique étant déjà présent pour la réaction d'hydrocyanation du butadiène, il suffit d'arrêter toute introduction de cyanure d'hydrogène, pour laisser se produire la réaction d'isomérisation.

On peut, le cas échéant, dans cette variante faire un léger balayage du réacteur à l'aide d'un gaz inerte comme l'azote ou l'argon par exemple, afin de chasser l'acide cyanhydrique qui pourrait être encore présent.

La réaction d'isomérisation est généralement réalisée à une température de 10°C à 200°C et de préférence de 60°C à 120°C.

Dans le cas préféré d'une isomérisation suivant immédiatement la réaction d'hydrocyanation du butadiène, il sera avantageux d'opérer à la température à laquelle l'hydrocyanation a été conduite.

Comme pour le procédé d'hydrocyanation de composés à insaturation éthylénique, la solution catalytique utilisée pour l'isomérisation peut être préparée avant son introduction dans la zone de réaction, par exemple par addition à la solution aqueuse de la phosphine hydrosoluble de formule (I) ou (II), de la quantité appropriée de composé du métal de transition choisi et éventuellement du réducteur. Il est également possible de préparer la solution catalytique "in situ" par simple mélange de ces divers constituants. La quantité de composé du métal de transition et plus particulièrement du nickel utilisée, ainsi que la quantité de phosphine hydrosoluble de formule (I) ou (II) sont les mêmes que pour la réaction d'hydrocyanation.

Bien que la réaction d'isomérisation soit conduite généralement sans tiers-solvant, il peut être avantageux de rajouter un solvant organique inerte, non miscible à l'eau, qui pourra être celui de l'extraction ultérieure. C'est notamment le cas lorsqu'un tel solvant a été mis en oeuvre dans la réaction d'hydrocyanation du butadiène ayant servi à préparer le milieu soumis à la réaction d'isomérisation. De tels solvants peuvent être choisis parmi ceux qui ont été cités précédemment pour l'hydrocyanation.

En fin de réaction, il est très aisé de séparer le catalyseur des produits de la réaction d'isomérisation comme cela a été indiqué pour l'hydrocyanation et de le recycler le cas échéant dans l'une des réactions d'hydrocyanation décrites précédemment ou dans une nouvelle réaction d'isomérisation.

Les exemples qui suivent illustrent la présente invention.

### EXEMPLE 1

### 1) Préparation de la solution catalytique Ni/CBDTSNa4.

Dans un ballon en verre de 100 ml, muni d'un barreau aimanté et d'un réfrigérant ascendant, on charge 50 ml d'une solution de 11,3 mmol de sel de sodium
du 1,2-bis[di(sulfonatophényl)phosphinométhyl] cyclobutane (CBDTSNa4) dans l'eau ; on dégaze cette solution. On introduit ensuite, sous agitation et sous courant d'argon, 2 g (7,3 mmol) de Ni(cyclooctadiène)₂, puis 35 ml d'ortho-xylène préalablement dégazé.

On chauffe à 45°C pendant 15 h. Après refroidissement, le système biphasique est décanté et la phase aqueuse fortement colorée en rouge est prélevée.

L'analyse élémentaire de la phase aqueuse révèle une concentration en Ni de 8 mmol/100 g et en P de 35,5 mmol/100 g.

### 2) Hydrocyanation du pentène-3 nitrile.

Dans un réacteur en verre de 150 ml, agité à l'aide d'une turbine, on charge 37,4 g de la solution aqueuse préparée en 1). On chauffe sous agitation à 60°C, puis en maintenant cette température on injecte successivement :
- 3,2 ml d'une solution aqueuse contenant 20 mmol de chlorure de Zn
- 16,5 g (204 mmol) de pentène-3 nitrile (3PN).

On injecte ensuite du cyanure d'hydrogène à raison de 1,2 g/h (44mmol/h) pendant 0,5 h.

En fin d'essai, on refroidit le mélange réactionnel obtenu, on neutralise à l'aide d'une solution concentrée de soude l'éventuel excès de cyanure d'hydrogène injecté et on dose les différents constituants par chromatographie en phase gazeuse (CPG).

On obtient les résultats suivants :
- taux de transformation (TT) du 3PN 7 %
- rendement (RT) en adiponitrile (ADN) par rapport
   au 3PN transformé 81 %
- RT en méthyl-2 glutaronitrile (MGN) par rapport
   au 3PN transformé 10 %
- RT en éthyl-2 succinonitrile (ESN) par rapport
   au 3PN transformé 1 %
- RT en valéronitrile (VN) par rapport au 3PN transformé 8 %
- linéarité(*) 89 %
- activité du catalyseur (**) 4
- productivité en ADN (rapportée au volume
   de la phase aqueuse) 65 g/h.l
(^{*}) ADN formé/ADN + MGN + ESN formés
(^{**}) nombre de moles de 3PN transformé par mole de Ni engagé

### EXEMPLE 2

### 1) Préparation de la solution catalytique Ni/TPPPNa6.

Dans un ballon en verre de 100 ml, muni d'un barreau aimanté et d'un réfrigérant ascendant, on charge 50 ml d'une solution de 32,8 mmol de sel de sodium de la tris(paraphosphophényl) phosphine (TPPPNa6) dans l'eau ; on dégaze cette solution. On introduit ensuite, sous agitation et sous courant d'argon, 2 g (7,3 mmol) de Ni(cyclooctadiène)₂, puis 35 ml d'ortho-xylène préalablement dégazé.

On chauffe à 45°C pendant 15 h. Après refroidissement, le système biphasique est décanté et la phase aqueuse fortement colorée en rouge est prélevée.

L'analyse élémentaire de la phase aqueuse révèle une concentration en Ni de 11,9 mmol/100 g et en P de 216,2 mmol/100 g.

### 2) Hydrocyanation du pentène-3 nitrile.

Dans un réacteur en verre de 150 ml, agité à l'aide d'une turbine, on charge 42,0 g de la solution aqueuse préparée en 1). On chauffe sous agitation à 60°C, puis en maintenant cette température on injecte successivement :
- 3,2 ml d'une solution aqueuse contenant 20 mmol de chlorure de Zn
- 23,5 g (290 mmol) de pentène-3 nitrile (3PN).

On injecte ensuite du cyanure d'hydrogène à raison de 1,8 g/h (67mmol/h) pendant 0,6 h.

En fin d'essai, on refroidit le mélange réactionnel obtenu, on neutralise à l'aide d'une solution concentrée de soude l'éventuel excès de cyanure d'hydrogène injecté et on dose les différents constituants par chromatographie en phase gazeuse (CPG).

On obtient les résultats suivants :
- taux de transformation (TT) du 3PN 13 %
- rendement (RT) en adiponitrile (ADN) par rapport
   au 3PN transformé 70 %
- RT en méthyl-2 glutaronitrile (MGN) par rapport
   au 3PN transformé 23%
- RT en éthyl-2 succinonitrile (ESN) par rapport
   au 3PN transformé 2 %
- RT en valéronitrile (VN) par rapport au 3PN transformé 4 %
- linéarité 73 %
- activité du catalyseur 7
- productivité en ADN (rapportée au volume
   de la phase aqueuse) 125 g/h.l

### EXEMPLE 3

### 1) Préparation de la solution catalytique Ni/DSPCPPNa3.

Dans un ballon en verre de 100 ml, muni d'un barreau aimanté et d'un réfrigérant ascendant, on charge 50 ml d'une solution de 32,8 mmol de sel de sodium
de la bis(métasulfophényl) paracarboxyphényl phosphine (DSPCPPNa3) dans l'eau ; on dégaze cette solution. On introduit ensuite, sous agitation et sous courant d'argon, 2 g (7,3 mmol) de Ni(cyclooctadiène)₂, puis 35 ml d'ortho-xylène préalablement dégazé.

On chauffe à 45°C pendant 15 h. Après refroidissement, le système biphasique est décanté et la phase aqueuse fortement colorée en rouge est prélevée.

L'analyse élémentaire de la phase aqueuse révèle une concentration en Ni de 12 mmol/100 g et en P de 53,9 mmol/100 g.

### 2) Hydrocyanation du pentène-3 nitrile.

Dans un réacteur en verre de 150 ml, agité à l'aide d'une turbine, on charge 41,7 g de la solution aqueuse préparée en 1). On chauffe sous agitation à 60°C, puis en maintenant cette température on injecte successivement :
- 3,2 ml d'une solution aqueuse contenant 20 mmol de chlorure de Zn
- 23,5 g (290 mmol) de pentène-3 nitrile (3PN).

On injecte ensuite du cyanure d'hydrogène à raison de 1,8 g/h (67mmol/h) pendant 0,75 h.

En fin d'essai, on refroidit le mélange réactionnel obtenu, on neutralise à l'aide d'une solution concentrée de soude l'éventuel excès de cyanure d'hydrogène injecté et on dose les différents constituants par chromatographie en phase gazeuse (CPG).

On obtient les résultats suivants:
- taux de transformation (TT) du 3PN 19 %
- rendement (RT) en adiponitrile (ADN) par rapport
   au 3PN transformé 71 %
- RT en méthyl-2 glutaronitrile (MGN) par rapport
   au 3PN transformé 20 %
- RT en éthyl-2 succinonitrile (ESN) par rapport
   au 3PN transformé 3 %
- RT en valéronitrile (VN) par rapport au 3PN transformé 6 %
- linéarité 76 %
- activité du catalyseur 10
- productivité en ADN (rapportée au volume
   de la phase aqueuse) 150 g/h.l

### EXEMPLE 4

### 1) Préparation de la solution catalytique Ni/DSPSEPNa3.

Dans un ballon en verre de 100 ml, muni d'un barreau aimanté et d'un réfrigérant ascendant, on charge 50 ml d'une solution de 32,8 mmol de sel de sodium
de la bis(métasulfophényl) sulfo-2 éthyl phosphine (DSPSEPNa3) dans l'eau ; on dégaze cette solution. On introduit ensuite, sous agitation et sous courant d'argon, 2 g (7,3 mmol) de Ni(cyclooctadiène)₂, puis 35 ml d'ortho-xylène préalablement dégazé.

On chauffe à 45°C pendant 15 h. Après refroidissement, le système biphasique est décanté et la phase aqueuse fortement colorée en rouge est prélevée.

L'analyse élémentaire de la phase aqueuse révèle une concentration en Ni de 11,8 mmol/100 g et en P de 54,5 mmol/100 g.

### 2) Hydrocyanation du pentène-3 nitrile.

Dans un réacteur en verre de 150 ml, agité à l'aide d'une turbine, oncharge 42,4 g de la solution aqueuse préparée en 1). On chauffe sous agitation à 60°C, puis en maintenant cette température on injecte successivement :
- 3,2 ml d'une solution aqueuse contenant 20 mmol de chlorure de Zn
- 23,5 g (290 mmol) de pentène-3 nitrile (3PN).

On injecte ensuite du cyanure d'hydrogène à raison de 1,8 g/h (67mmol/h) pendant 0,5 h.

En fin d'essai, on refroidit le mélange réactionnel obtenu, on neutralise à l'aide d'une solution concentrée de soude l'éventuel excès de cyanure d'hydrogène injecté et on dose les différents constituants par chromatographie en phase gazeuse (CPG).

On obtient les résultats suivants :
- taux de transformation (TT) du 3PN 8 %
- rendement (RT) en adiponitrile (ADN) par rapport
   au 3PN transformé 69 %
- RT en méthyl-2 glutaronitrile (MGN) par rapport
   au 3PN transformé 24 %
- RT en éthyl-2 succinonitrile (ESN) par rapport
   au 3PN transformé 3 %
- RT en valéronitrile (VN) par rapport au 3PN transformé 4 %
- linéarité 72 %
- activité du catalyseur 4
- productivité en ADN (rapportée au volume
   de la phase aqueuse) 90 g/h.l

### ESSAI COMPARATIF

### 1) Préparation de la solution catalytigue Ni/TPPTSNa3.

Dans un ballon en verre de 1000 ml, muni d'un agitateur et d'un réfrigérant ascendant, on charge 500 ml d'une solution de 300 mmol de sel de sodium de la tris(métasulfophényl) phosphine (TPPTSNa3) dans l'eau ; on dégaze cette solution. On introduit ensuite, sous agitation et sous courant d'argon, 20 g (73 mmol) de Ni(cyclooctadiène)₂, puis 350 ml d'ortho-xylène préalablement dégazé.

On chauffe à 45°C pendant 15 h. Après refroidissement, le système biphasique est décanté et la phase aqueuse fortement colorée en rouge est prélevée.

L'analyse élémentaire de la phase aqueuse révèle une concentration en Ni de 12,0 mmol/100 g et en P de 49,7 mmol/100 g.

### 2) Hydrocyanation du pentène-3 nitrile.

Dans un réacteur en verre de 150 ml, agité à l'aide d'une turbine, on charge 41,7 g de la solution aqueuse préparée en 1). On chauffe sous agitation à 60°C, puis en maintenant cette température on injecte successivement :
- 3,2 ml d'une solution aqueuse contenant 20 mmol de chlorure de Zn
- 8 g (105 mmol) de pentène-3 nitrile (3PN).

On injecte ensuite du cyanure d'hydrogène à raison de 1,8 g/h (67mmol/h) pendant 2 h.

En fin d'essai, on refroidit le mélange réactionnel obtenu, on neutralise à l'aide d'une solution concentrée de soude l'éventuel excès de cyanure d'hydrogène injecté et on dose les différents constituants par chromatographie en phase gazeuse (CPG).

On obtient les résultats suivants :
- taux de transformation (TT) du 3PN 89 %
- rendement (RT) en adiponitrile (ADN) par rapport
   au 3PN transformé 66 %
- RT en méthyl-2 glutaronitrile (MGN) par rapport
   au 3PN transformé 26 %
- RT en éthyl-2 succinonitrile (ESN) par rapport
   au 3PN transformé 5 %
- RT en valéronitrile (VN) par rapport au 3PN transformé 3 %
- linéarité 68 %
- activité du catalyseur 20
- productivité en ADN (rapportée au volume
   de la phase aqueuse) 90 g/h.l

## Revendications

1. Procédé d'hydrocyanation de composés organiques comportant au moins une double liaison éthylénique par réaction avec le cyanure d'hydrogène, en présence d'une solution aqueuse d'un catalyseur comprenant au moins un composé d'un métal de transition et au moins une phosphine hydrosoluble, caractérisé en ce que ladite phosphine hydrosoluble est une phosphine monodentate ou bidentate répondant à la formule générale (I): 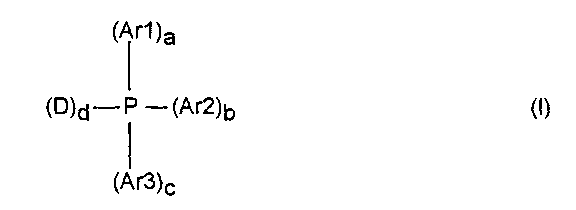 dans laquelle :
- Ar1 et Ar2, identiques ou différents, représentent des groupes aryle ou aryle comportant un ou plusieurs substituants tels que :
- radical alkyle ou alcoxy ayant 1 à 4 atomes de carbone,
- atome d'halogène,
- groupe hydrophile comme :
- COOM, -SO₃M, -PO₃M, M représentant un reste cationique minéral ou organique choisi parmi le proton, les cations dérivés des métaux alcalins ou alcalino-terreux, les cations ammonium -N(R)₄ dans la formule desquels les symboles R, identiques ou différents, représentent un atome d'hydrogène ou un radical alkyle ayant de 1 à 4 atomes de carbone, les autres cations dérivés de métaux dont les sels des acides arylcarboxyliques, des acides arylsulfoniques ou des acides arylphosphoniques sont solubles dans l'eau,
- N(R)₃ dans la formule duquel les symboles R, identiques ou différents, représentent un atome d'hydrogène ou un radical alkyle ayant de 1 à 4 atomes de carbone,
- OH
- Ar3 représente un groupe aryle comportant un ou plusieurs substituants tels que :
- radical alkyle ou alcoxy ayant 1 à 4 atomes de carbone,
- atome d'halogène,
- groupe hydrophile comme :
- COOM, -PO₃M, M représentant un reste cationique minéral ou organique choisi parmi le proton, les cations dérivés des métaux alcalins ou alcalino-terreux, les cations ammonium -N(R)₄ dans la formule desquels les symboles R, identiques ou différents, représentent un atome d'hydrogène ou un radical alkyle ayant de 1 à 4 atomes de carbone, les autres cations dérivés de métaux dont les sels des acides arylcarboxyliques ou des acides arylphosphoniques sont solubles dans l'eau,
l'un au moins desdits substituants de Ar3 étant un groupe hydrophile tel que défini précédemment,
- a représente 0 ou 1,
- b représente 0 ou 1,
- c représente un nombre entier de 0 à 3,
- D représente un groupe alkyle, un groupe cycloalkyle, un groupe alkyle ou cycloalkyle comportant un ou plusieurs substituants tels que :
- radical alcoxy ayant 1 à 4 atomes de carbone,
- atome d'halogène,
- groupe hydrophile comme :
- COOM, -SO₃M, -PO₃M, M représentant un reste cationique minéral ou organique choisi parmi le proton, les cations dérivés des métaux alcalins ou alcalino-terreux, les cations ammonium -N(R)₄ dans la formule desquels les symboles R, identiques ou différents, représentent un atome d'hydrogène ou un radical alkyle ayant de 1 à 4 atomes de carbone, les autres cations dérivés de métaux dont les sels des acides arylcarboxyliques, des acides arylsulfoniques ou des acides arylphosphoniques sont solubles dans l'eau,
- N(R)₃ dans la formule duquel les symboles R, identiques ou différents, représentent un atome d'hydrogène ou un radical alkyle ayant de 1 à 4 atomes de carbone,
- OH
- d représente un nombre entier de 0 à 3,
- la somme (a+b+c+d) est égale à 3
ou à la formule générale (II) : 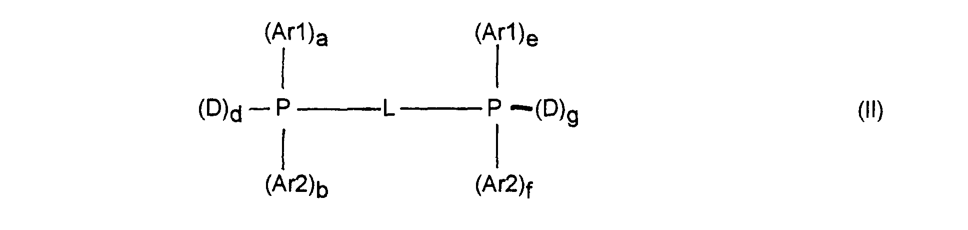 dans laquelle :
- Ar1, Ar2 et D ont les significations indiquées précédemment pour la formule (I),
- a, b, e et f représentent chacun 0 ou 1,
- d et g représentent chacun un nombre entier de 0 à 2,
- la somme (a+b+d) est égale à 2,
- la somme (e+f+g) est égale à 2,
- L représente un lien valentiel simple ou un radical hydrocarboné divalent tel qu'un radical alkylène, un radical cycloalkylène, un radical phénylène, un radical diphénylène, un radical dérivant d'un hétérocycle comportant un ou deux atomes d'oxygène, d'azote ou de soufre dans le cycle, ces différents radicaux cycliques étant liés directement à l'un des atomes de phosphore ou aux deux atomes de phosphore ou étant lié à l'un des atomes de phosphore ou aux deux par l'intermédiaire d'un radical alkylène linéaire ou ramifié ayant de 1 à 4 atomes de carbone, le ou les cycles éventuellement parties du radical divalent L pouvant comporter un ou plusieurs substituants tels que groupe alkyle ayant 1 à 4 atomes de carbone.

2. Procédé selon la revendication 1, caractérisé en ce que les phosphines hydrosolubles sont les phosphines de formule (I) ou de formule (II) dans lesquelles Ar1 et Ar2 sont des groupes phényle ou des groupes phényles comportant un ou deux substituants tels que définis précédemment, Ar3 est un groupe phényle comportant un ou deux substituants tels que définis précédemment, D est groupe alkyle ayant de 1 à 6 atomes de carbone, un groupe cycloalkyle ayant 5 à 8 atomes de carbone, un groupe alkyle ayant de 1 à 6 atomes de carbone ou cycloalkyle ayant 5 à 8 atomes de carbone comportant un ou plusieurs substituants tels que définis précédemment, L est un lien valentiel simple, un radical alkylène ayant de 1 à 6 atomes de carbone, un radical cycloalkylène monocyclique ou bicyclique ayant de 4 à 12 atomes de carbone, un radical phénylène, un radical diphénylène, un radical dérivant d'un hétérocycle comportant un ou deux atomes d'oxygène, d'azote ou de soufre dans le cycle, ces différents radicaux cycliques étant liés directement à l'un des atomes de phosphore ou aux deux atomes de phosphore ou étant lié à l'un des atomes de phosphore ou aux deux par l'intermédiaire d'un radical alkylène linéaire ou ramifié ayant de 1 à 4 atomes de carbone, le ou les cycles éventuellement parties du radical divalent L pouvant comporter un ou plusieurs substituants tels que groupe alkyle ayant 1 à 4 atomes de carbone.

3. Procédé selon l'une des revendications 1 ou 2, caractérisé en ce que les phosphines hydrosolubles sont les phosphines de formule (I) ou de formule (II) dans lesquelles:
- le ou les substituants de Ar1 et Ar2, identiques ou différents, représentent des groupes tels que :
- radical alkyle ou alcoxy ayant 1 à 2 atomes de carbone,
- atome de chlore,
- groupe hydrophile comme :
- COOM, -SO₃M, -PO₃M, M représentant un reste cationique minéral ou organique choisi parmi le proton, les cations dérivés du sodium, du potassium, du calcium ou du baryum, les cations ammonium, tétraméthylammonium, tétraéthylammonium, tétrapropylammonium, tétrabutylammonium, les cations dérivés du zinc, du plomb ou de l'étain,
- N(R)₃ dans la formule duquel les symboles R, identiques ou différents, représentent un atome d'hydrogène ou un radical alkyle ayant de 1 à 4 atomes de carbone,
- OH
- le ou les substituants de Ar3, identiques ou différents, représentent des groupes tels que :
- radical alkyle ou alcoxy ayant 1 à 2 atomes de carbone,
- atome de chlore,
- groupe hydrophile comme :
- COOM, -PO₃M, M représentant un reste cationique minéral ou organique choisi parmi le proton, les cations dérivés du sodium, du potassium, du calcium ou du baryum, les cations ammonium, tétraméthylammonium, tétraéthylammonium, tétrapropylammonium, tétrabutylammonium, les cations dérivés du zinc, du plomb ou de l'étain,
globalement deux au moins desdits substituants de Ar1, Ar2, Ar3 et D pour les phosphines de formule (I) et de Ar1, Ar2 et D pour les phosphines de formule (II) étant un groupe hydrophile tel que défini précédemment.

4. Procédé selon l'une des revendications 1 à 3, caractérisé en ce que les composés des métaux de transition sont choisis parmi les composés du nickel, du palladium et du fer, solubles dans l'eau ou capables de passer en solution dans les conditions de la réaction.

5. Procédé selon l'une des revendications 1 à 4, caractérisé en ce que les composés des métaux de transition préférés sont ceux du nickel et sont choisis parmi :
- les composés dans lesquel le nickel est au degré d'oxydation zéro comme le tétracyanonickelate de potassium K₄ [(Ni(CN)₄], le bis(acrylonitrile) nickel zéro,
le bis(cyclooctadiène-1,5)₂ nickel et les dérivés contenant des ligands du groupe Va comme le tétrakis(triphényl-phosphine) nickel zéro ;
- les composés du nickel comme les carboxylates, carbonate, bicarbonate, borate, bromure, chlorure, citrate, thiocyanate, cyanure, formiate, hydroxyde, hydrophosphite, phosphite, phosphate et dérivés, iodure, nitrate, sulfate, sulfite, aryl- et alkyl-sulfonates.

6. Procédé selon l'une des revendications 1 à 5, caractérisé en ce que les composés organiques comportant au moins une double liaison éthylénique sont choisis parmi les dioléfines comme le butadiène, l'isoprène, l'hexadiène-1,5, le cyclooctadiène-1,5, les nitriles aliphatiques à insaturation éthylénique, particulièrement les pentène-nitriles linéaires comme le pentène-3 nitrile, le pentène-4 nitrile, les monooléfines comme le styrène, le méthyl-styrène, le vinyl-naphtalène, le cyclohexène, le méthyl-cyclohexène ainsi que les mélanges de plusieurs de ces composés.

7. Procédé selon l'une des revendications 1 à 6, caractérisé en ce que la quantité de composé du nickel ou d'un autre métal de transition utilisée est choisie de telle sorte qu'il y ait par litre de solution réactionnelle entre 10⁻⁴ et 1, et de préférence entre 0,005 et 0,5 mole de nickel ou de l'autre métal de transition mis en oeuvre et en ce que la quantité de phosphine hydrosoluble de formule (I) ou (II) utilisée est choisie de telle sorte que le nombre de moles de ce composé rapporté à 1 mole de métal de transition soit de 0,5 à 2000 et de préférence de 2 à 300.

8. Procédé selon l'une des revendications 1 à 7, caractérisé en ce que la réaction d'hydrocyanation est réalisée à une température de 10°C à 200°C et de préférence de 30°C à 120°C.

9. Procédé selon l'une des revendications 1 à 8 d'hydrocyanation de composés à insaturation éthylénique, par réaction avec le cyanure d'hydrogène, caractérisé en ce que l'on opère en présence d'une solution aqueuse d'un catalyseur comprenant au moins un composé d'un métal de transition, au moins une phosphine hydrosoluble de formule (I) ou (II) et un cocatalyseur consistant en au moins un acide de Lewis.

10. Procédé selon la revendication 9, caractérisé en ce les composés à insaturation éthylénique sont choisis parmi les nitriles aliphatiques à insaturation éthylénique, de préférence les pentène-nitriles linéaires comme le pentène-3 nitrile, le pentène-4 nitrile et leurs mélanges.

11. Procédé selon la revendication 10, caractérisé en ce que les pentène-nitriles linéaires contiennent des quantités, généralement minoritaires, d'autres composés, comme le méthyl-2 butène-3 nitrile, le méthyl-2 butène-2 nitrile, le pentène-2 nitrile,
le valéronitrile, l'adiponitrile, le méthyl-2 glutaronitrile, l'éthyl-2 succinonitrile ou le butadiène.

12. Procédé selon l'une des revendications 9 à 11, caractérisé en ce que l'acide de Lewis mis en oeuvre comme cocatalyseur est choisi parmi les composés des éléments des groupes Ib, IIb, IIIa, IIIb, IVa, IVb, Va, Vb, VIb, VIIb et VIII de la Classification périodique des éléments, dans la mesure où lesdits composés sont au moins partiellement solubles et stables dans l'eau.

13. Procédé selon l'une des revendications 9 à 12, caractérisé en ce que l'acide de Lewis est choisi parmi les sels, notamment des halogénures, des sulfates,
des carboxylates et des phosphates.

14. Procédé selon l'une des revendications 9 à 13, caractérisé en ce que l'acide de Lewis est choisi parmi le chlorure de zinc, le bromure de zinc, l'iodure de zinc,
le chlorure de manganèse, le bromure de manganèse, le chlorure de cadmium,
le bromure de cadmium, le chlorure stanneux, le bromure stanneux, le sulfate stanneux, le tartrate stanneux, les chlorures ou bromures des éléments des terres rares comme le lanthane, le cérium, le praséodyme, le néodyme, le samarium, l'europium, le gadolinium, le terbium, le dysprosium, l'holmium, l'erbium, le thulium, l'ytterbium et le lutétium,
le chlorure de cobalt, le chlorure ferreux, le chlorure d'yttrium et leurs mélanges.

15. Procédé selon l'une des revendications 9 à 14, caractérisé en ce que l'acide de Lewis mis en oeuvre représente de 0,01 à 50 moles par mole de composé de métal de transition, plus particulièrement de composé du nickel, et de préférence de 1 à 10 mole par mole.

16. Procédé selon l'une des revendications 1 à 15, caractérisé en ce que l'on réalise en absence de cyanure d'hydrogène l'isomérisation en pentène-nitriles du méthyl-2 butène-3 nitrile présent dans le mélange réactionnel provenant de l'hydrocyanation du butadiène, en opérant en présence d'un catalyseur comportant au moins une phosphine hydrosoluble de formule (I) ou (II) et au moins un composé d'un métal de transition.

17. Procédé selon la revendication 16, caractérisé en ce que le méthyl-2 butène-3 nitrile soumis à l'isomérisation est mis en oeuvre seul ou en mélange avec du méthyl-2 butène-2 nitrile, du pentène-4 nitrile, du pentène-3 nitrile, du pentène-2 nitrile, du butadiène, de l'adiponitrile, du méthyl-2 glutaroronitrile, de l'éthyl-2 succinonitrile ou du valéronitrile.

18. Procédé selon l'une des revendications 16 ou 17, caractérisé en ce que la réaction d'isomérisation est réalisée à une température de 10°C à 200°C et de préférence de 60°C à 120°C.

19. Procédé selon l'une des revendications 16 à 18, caractérisé en ce que l'isomérisation en pentène-nitriles du méthyl-2 butène-3 nitrile est réalisée en présence d'une solution aqueuse d'un catalyseur comprenant au moins un composé d'un métal de transition, au moins une phosphine hydrosoluble de formule (I) ou (II) et un cocatalyseur consistant en au moins un acide de Lewis.

## Patentansprüche

1. Verfahren zur Hydrocyanierung von organischen Verbindungen, die zumindest eine ethylenische Doppelbindung enthalten, durch Umsetzung mit Cyanwasserstoff in Anwesenheit einer wäßrigen Lösung eines Katalysators, umfassend zumindest eine Verbindung eines Übergangsmetalls und zumindest ein wasserlösliches Phosphin, dadurch gekennzeichnet, daß das wasserlösliche Phosphin ein einzähniges oder zweizähniges Phosphin ist der allgemeinen Formel (I) 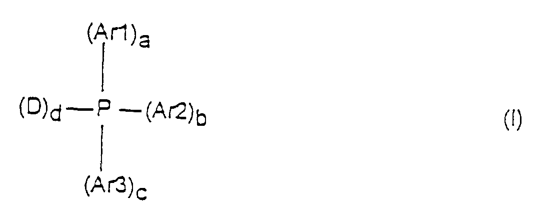 worin
- Ar1 und Ar2, identisch oder verschieden, Arylgruppen oder Arylgruppen, enthaltend einen oder mehrere Substituenten, wie:
- Alkyl- oder Alkoxyrest mit 1 bis 4 Kohlenstoffatomen,
- Halogenatom,
- hydrophile Gruppe, wie:
- COOM, -SO₃M, -PO₃M, wobei M einen anorganischen oder organischen kationischen Rest bedeutet, ausgewählt unter dem Proton, den sich von Alkali- oder Erdalkalimetallen ableitenden Kationen, den Ammoniumkationen -N(R)₄, in deren Formel die Symbole R, identisch oder verschieden, ein Wasserstoffatom oder einen Alkylrest mit 1 bis 4 Kohlenstoffatomen bedeuten, den anderen, sich von Metallen ableitenden Kationen, deren Salze der Arylcarbonsäuren, der Arylsulfonsäuren oder der Arylphosphonsäuren in Wasser löslich sind,
- N(R)₃, in dessen Formel die Symbole R, identisch oder verschieden, ein Wasserstoffatom oder einen Alkylrest mit 1 bis 4 Kohlenstoffatomen bedeuten,
- OH,
bedeuten,
- Ar3 eine Arylgruppe mit einem oder mehreren Substituenten wiedergibt, wie:
- Alkyl- oder Alkoxyrest mit 1 bis 4 Kohlenstoffatomen,
- Halogenatom,
- hydrophile Gruppe, wie:
- COOM, -PO₃M, wobei M einen anorganischen oder organischen kationischen Rest bedeutet, ausgewählt unter dem Proton, den sich von Alkali- oder Erdalkalimetallen ableitenden Kationen, den Ammoniumionen -N(R)₄, in deren Formel die Symbole R, identisch oder verschieden, ein Wasserstoffatom oder einen Alkylrest mit 1 bis 4 Kohlenstoffatomen bedeuten, den anderen, sich von Metallen ableitenden Kationen, deren Salze der Arylcarbonsäuren oder der Arylphosphonsäuren in Wasser löslich sind, wobei zumindest einer der Substituenten von Ar3 eine hydrophile Gruppe ist, wie zuvor definiert,
- a für 0 oder 1 steht,
- b für 0 oder 1 steht,
- c eine ganze Zahl von 0 bis 3 bedeutet,
- D eine Alkylgruppe, eine Cycloalkylgruppe, eine Alkyl- oder Cycloalkylgruppe mit einem oder mehreren Substituenten, wie:
- Alkoxyrest mit 1 bis 4 Kohlenstoffatomen,
- Halogenatom,
- hydrophile Gruppe, wie:
- COOM, -SO₃M, -PO₃M, wobei M ein anorganischer oder organischer kationischer Rest ist, ausgewählt unter dem Proton, den Kationen, abgeleitet von Alkali- oder Erdalkalimetallen, den Ammoniumkationen -N(R)₄, in deren Formel die Symbole R, identisch oder verschieden, ein Wasserstoffatom oder einen Alkylrest mit 1 bis 4 Kohlenstoffatomen bedeuten, den anderen, sich von Metallen ableitenden Kationen, deren Salze der Arylcarbonsäuren, Arylsulfonsäuren oder Arylphosphonsä uren in Wasser löslich sind,
- N(R)₃, in dessen Formel die Symbole R, identisch oder verschieden, ein Wasserstoffatom oder einen Alkylrest mit 1 bis 4 Kohlenstoffatomen bedeuten,
- OH
wiedergibt,
- d eine ganze Zahl von 0 bis 3 bedeutet,
- die Summe (a+b+c+d) 3 entspricht,
oder der allgemeinen Formel (II) 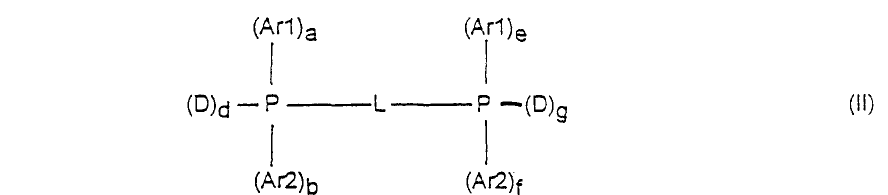 worin
- Ar1, Ar2 und D die vorstehend für die Formel (I) angegebenen Bedeutungen besitzen,
- a, b, e und f jeweils für 0 oder 1 stehen,
- d und g jeweils eine ganze Zahl von 0 bis 2 wiedergeben,
- die Summe (a+b+d) 2 entspricht,
- die Summe (e+f+e) 2 entspricht,
- L eine einfache Bindung oder einen zweiwertigen Kohlenwasserstoffrest, wie einen Alkylenrest, einen Cycloalkylenrest, einen Phenylenrest, einen Diphenylenrest, einen Rest, abgeleitet von einem Heterocyclus mit einem oder zwei Sauerstoff-, Stickstoff- oder Schwefelatomen in dem Ring, wobei diese verschiedenen cyclischen Reste direkt an eines der Phosphoratome oder an zwei Phosphoratome gebunden sind oder an einen der Phosphoratome oder an zwei über einen linearen oder verzweigten Alkylenrest mit 1 bis 4 Kohlenstoffatomen gebunden sind, wobei der oder die Ringe, die gegebenenfalls einen Teil des zweiwertigen Restes L bilden, einen oder mehrere Substituenten, wie eine Alkylgruppe mit 1 bis 4 Kohlenstoffatomen, aufweisen können, bedeutet.

2. Verfahren gemäß Anspruch 1, dadurch gekennzeichnet, daß die wasserlöslichen Phosphine Phosphine der Formel (I) oder der Formel (II) sind, worin Ar1 und Ar2 Phenylgruppen oder Phenylgruppen, enthaltend einen oder zwei Substituenten, wie vorstehend definiert bedeuten, Ar3 eine Phenylgruppe, enthaltend einen oder zwei Substituenten, wie vorstehend definiert, wiedergibt, D eine Alkylgruppe mit 1 bis 6 Kohlenstoffatomen, eine Cycloalkylgruppe mit 5 bis 8 Kohlenstoffatomen, eine Gruppe Alkyl mit 1 bis 6 Kohlenstoffatomen oder Cycloalkyl mit 5 bis 8 Kohlenstoffatomen, enthaltend einen oder mehrere Substituenten, wie vorstehend definiert, ist, L eine Einfachbindung, einen Alkylenrest mit 1 bis 6 Kohlenstoffatomen, einen monocyclischen oder bicyclischen Cycloalkylenrest mit 4 bis 12 Kohlenstoffatomen, einen Phenylenrest, einen Diphenylenrest, einen Rest, der sich von einem Heterocyclus mit 1 oder 2 Sauerstoff-, Stickstoff- oder Schwefelatomen im Ring ableitet, bedeutet, wobei diese verschiedenen cyclischen Reste direkt an eines der Phosphoratome oder zwei der Phosphoratome gebunden sind oder an eines der Phosphoratome oder zwei über einen linearen oder verzweigten Alkylenrest mit 1 bis 4 Kohlenstoffatomen gebunden sind, wobei der oder die Ringe, die gegebenenfalls einen Teil des zweiwertigen Restes L bilden, einen oder mehrere Substituenten, wie eine Alkylgruppe mit 1 bis 4 Kohlenstoffatomen, aufweisen können.

3. Verfahren gemäß Anspruch 1 oder 2, dadurch gekennzeichnet, daß die wasserlöslichen Phosphine Phosphine der Formel
(I) oder der Formel (II) sind, worin:
- der oder die Substituenten von Ar1 und Ar2, identisch oder verschieden, Gruppen wiedergeben, wie:
- Alkyl- oder Alkoxyrest mit 1 bis 2 Kohlenstoffatomen,
- Chloratom,
- hydrophile Gruppe, wie:
- COOM, -SO₃M, -PO₃M, wobei M einen anorganischen oder organischen kationischen Rest, ausgewählt unter dem Proton, den sich von Natrium, Kalium, Calcium oder Barium ableitenden Kationen, den Ammonium-, Tetramethylammonium-, Tetraethylammonium-, Tetrapropylammonium-, Tetrabutylammoniumkationen, den sich von Zink, Blei oder Zinn ableitenden Kationen, bedeutet,
- N(R)₃, in dessen Formel die Symbole R, identisch oder verschieden, ein Wasserstoffatom oder einen Alkylrest mit 1 bis 4 Kohlenstoffatomen wiedergeben,
- OH,
- der oder die Substituenten von Ar3, identisch oder verschieden, Gruppen bedeuten, wie:
- Alkyl- oder Alkoxyreste mit 1 bis 2 Kohlenstoffatomen,
- Chloratom,
- hydrophile Gruppe, wie:
- COOM, -PO₃M, wobei M einen anorganischen oder organischen kationischen Rest, ausgewählt unter dem Proton, den sich von Natrium, Kalium, Calcium oder Barium ableitenden Kationen, den Ammonium-, Tetrame thylammonium-, Tetraethylammonium-, Tetrapropylammonium-, Tetrabutylammoniumkationen, den sich von Zink, Blei oder Zinn ableitenden Kationen, bedeutet,
wobei insgesamt zumindest zwei der Substituenten von Ar1, Ar2, Ar3 und D für die Phosphine der Formel (I) und Ar1, Ar2 und D für die Phosphine der Formel (II) eine hydrophile Gruppe, wie vorstehend definiert, wiedergeben.

4. Verfahren gemäß einem der Ansprüche 1 bis 3, dadurch gekennzeichnet, daß die Übergangsmetallverbindungen unter den Verbindungen von Nickel, Palladium und Eisen, die in Wasser löslich sind oder befähigt sind, unter den Reaktionsbedingungen in Lösung zu gehen, ausgewählt werden.

5. Verfahren gemäß einem der Ansprüche 1 bis 4, dadurch gekennzeichnet, daß die bevorzugten Übergangsmetallverbindungen diejenigen des Nickels sind und ausgewählt werden unter:
- den Verbindungen, worin das Nickel sich im Oxidationszustand Null befindet, wie Kaliumtetracyanonickelat K₄[(Ni(CN)₄], Bis-(acrylnitril)-nickel-null, Bis-(1,5-cyclooctadien)₂-nickel und den Liganden der Gruppe Va aufweisenden Derivaten wie Tetrakis-(triphenyl-phosphin)-nickel-null;
- den Verbindungen des Nickels, wie die Carboxylate, Carbonat, Bicarbonat, Borat, Bromid, Chlorid, Citrat, Thiocyanat, Cyanid, Formiat, Hydroxid, Hydrophosphit, Phosphit, Phosphat und Derivate Jodid, Nitrat, Sulfat, Sulfit, Aryl- und Alkylsulfonate.

6. Verfahren gemäß einem der Ansprüche 1 bis 5, dadurch gekennzeichnet, daß die zumindest eine ethylenische Doppelbindung aufweisenden organischen Verbindungen ausgewählt werden unter den Diolefinen, wie Butadien, Isopren, 1,5-Hexadien, 1,5-Cyclooctadien, den aliphatischen Nitrilen mit ethylenischer Unsättigung, insbesondere den linearen Pentennitrilen, wie 3-Penten-nitril, 4-Penten-nitril, den Monoolefinen, wie Styrol, Methylstyrol, Vinylnaphthalin, Cyclohexen, Methylcyclohexen, sowie Gemische von mehreren dieser Verbindungen.

7. Verfahren gemäß einem der Ansprüche 1 bis 6, dadurch gekennzeichnet, daß die verwendete Menge an Verbindung von Nickel oder einem anderen Übergangsmetall derart ausgewählt wird, daß sie je Liter Reaktionsmischung zwischen 10⁻⁴ und 1 und vorzugsweise zwischen 0,005 und 0,5 Mol eingesetztes Nickel oder anderes Übergangsmetall beträgt,und daß die verwendete Menge des wasserlöslichen Phosphins der Formel (I) oder (II) derart ist, daß die Anzahl der Mole dieser Verbindung, bezogen auf 1 Mol Übergangsmetall 0,5 bis 2000 und vorzugsweise 2 bis 300 beträgt.

8. Verfahren gemäß einem der Ansprüche 1 bis 7, dadurch gekennzeichnet, daß die Hydrocyanierungsreaktion bei einer Temperatur von 10 bis 200°C und vorzugsweise 30 bis 120°C durchgeführt wird.

9. Verfahren gemäß einem der Ansprüche 1 bis 8 zur Hydrocyanierung von Verbindungen mit ethylenischer Unsättigung durch Umsetzung mit Cyanwasserstoff, dadurch gekennzeichnet, daß man in Anwesenheit einer wäßrigen Lösung eines Katalysators, umfassend zumindest eine Verbindung eines Übergangsmetalls, zumindest ein wasserlösliches Phosphin der Formel (I) oder (II) und einen Cokatalysator, bestehend aus zumindest einer Lewissäure, arbeitet.

10. Verfahren gemäß Anspruch 9, dadurch gekennzeichnet, daß die Verbindungen mit ethylenischer Unsättigung unter den aliphatischen Nitrilen mit ethylenischer Unsättigung, vorzugsweise den linearen Penten-nitrilen, wie 3-Penten-nitril, 4-Penten-nitril, und deren Gemischen ausgewählt werden.

11. Verfahren gemäß Anspruch 10, dadurch gekennzeichnet, daß die linearen Penten-nitrile Mengen, die im allgemeinen untergeordnet sind, an weiteren Verbindungen enthalten, wie 2-Methyl-3-buten-nitril, 2-Methyl-2-buten-nitril, 2-Penten-nitril, Valeronitril, Adiponitril, 2-Methyl-glutaronitril, 2-Ethyl-succinonitril oder Butadien.

12. Verfahren gemäß einem der Ansprüche 9 bis 11, dadurch gekennzeichnet, daß die als Cokatalysator eingesetzte Lewissäure unter den Verbindungen der Elemente der Gruppen Ib, IIb, IIIa, IIIb, IVa, IVb, Va, Vb, VIb, VIIb und VIII des Periodensystems der Elemente ausgewählt wird, mit der Maßgabe, daß besagte Verbindungen zumindest teilweise in Wasser löslich und stabil sind.

13. Verfahren gemäß einem der Ansprüche 9 bis 12, dadurch gekennzeichnet, daß die Lewissäure unter den Salzen, insbesondere den Halogeniden, den Sulfaten, den Carboxylaten und den Phosphaten, ausgewählt wird.

14. Verfahren gemäß einem der Ansprüche 9 bis 13, dadurch gekennzeichnet, daß die Lewissäure unter Zinkchlorid, Zinkbromid, Zinkjodid, Manganchlorid, Manganbromid, Cadmiumchlorid, Cadmiumbromid, Stannochlorid, Stannobromid, Stannosulfat, Stannotartrat, den Chloriden oder Bromiden der seltenen Erden, wie Lanthan, Cer, Praseodym, Neodym, Samarium, Europium, Gadolinium, Terbium, Dysprosium, Holmium, Erbium, Thulium, Ytterbium und Lutetium, dem Kobaltchlorid, dem Ferrochlorid, dem Yttriumchlorid und deren Gemischen, ausgewählt wird.

15. Verfahren gemäß einem der Ansprüche 9 bis 14, dadurch gekennzeichnet, daß die eingesetzte Lewissäure 0,01 bis 50 Mol je Mol Übergangsmetallverbindung, insbesondere Nickelverbindung, und vorzugsweise 1 bis 10 Mol je Mol ausmacht.

16. Verfahren gemäß einem der Ansprüche 1 bis 15, dadurch gekennzeichnet, daß man in Abwesenheit von Cyanwasserstoff die Isomerisierung zu Penten-nitrilen von 2-Methyl-3-butennitril durchführt, das in dem Reaktionsgemisch vorliegt, das der Hydrocyanierung von Butadien entstammt, wobei man in Gegenwart eines Katalysators arbeitet, der zumindest ein wasserlösliches Phosphin der Formel (I) oder (II) und zumindest eine Übergangsmetallverbindung enthält.

17. Verfahren gemäß Anspruch 16, dadurch gekennzeichnet, daß das der Isomerisierung unterzogene 2-Methyl-3-buten-nitril allein oder im Gemisch mit 2-Methyl-2-buten-nitril, 4-Pentennitril, 3-Penten-nitril, 2-Penten-nitril, Butadien, Adiponitril, 2-Methyl-glutaronitril, 2-Ethyl-succinonitril oder Valeronitril eingesetzt wird.

18. Verfahren gemäß einem der Ansprüche 16 oder 17, dadurch gekennzeichnet, daß die Isomerisierungsreaktion bei einer Temperatur von 10 bis 200°C, vorzugsweise von 60 bis 120°C, durchgeführt wird.

19. Verfahren gemäß einem der Ansprüche 16 bis 18, dadurch gekennzeichnet, daß die Isomerisierung zu Penten-nitrilen von 2-Methyl-3-buten-nitril in Anwesenheit einer wäßrigen Lösung eines Katalysators erfolgt, der zumindest eine Verbindung eines Übergangsmetalls, zumindest ein wasserlösliches Phosphin der Formel (I) oder (II) und einen Cokatalysator, bestehend aus zumindest einer Lewissäure, umfaßt.

## Claims

1. Process for the hydrocyanation of organic compounds containing at least one ethylenic double bond by reaction with hydrogen cyanide in the presence of an aqueous solution of a catalyst comprising at least one compound of a transition metal and at least one water-soluble phosphine, characterized in that the said water-soluble phosphine is a monodentate or bidentate phosphine corresponding to the general formula (I): 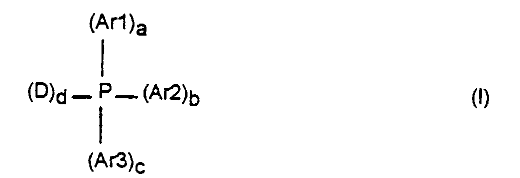 in which:
- Ar1 and Ar2, which are identical or different, represent aryl groups or aryl groups containing one or a number of substituents such as:
- alkyl or alkoxy radical having 1 to 4 carbon atoms,
- halogen atom,
- hydrophilic group, such as:
- COOM, -SO₃M or -PO₃M, M representing an inorganic or organic cationic residue chosen from the proton, cations derived from alkali or alkaline-earth metals, ammonium cations -N(R)₄, in the formula of which the R symbols, which are identical or different, represent a hydrogen atom or an alkyl radical having from 1 to 4 carbon atoms, and other cations derived from metals, the arylcarboxylic acid, arylsulphonic acid or arylphosphonic acid salts of which are soluble in water,
- N(R)₃, in the formula of which the R symbols, which are identical or different, represent a hydrogen atom or an alkyl radical having from 1 to 4 carbon atoms,
- OH
- Ar3 represents an aryl group containing one or a number of substituents, such as:
- alkyl or alkoxy radical having 1 to 4 carbon atoms,
- halogen atom,
- hydrophilic group, such as:
- COOM or -PO₃M, M representing an inorganic or organic cationic residue chosen from the proton, cations derived from alkali or alkaline-earth metals, ammonium cations -N(R)₄, in the formula of which the R symbols, which are identical or different, represent a hydrogen atom or an alkyl radical having from 1 to 4 carbon atoms, and other cations derived from metals, the arylcarboxylic acid or arylphosphonic acid salts of which are soluble in water,
at least one of the said substituents of Ar3 being a hydrophilic group as defined above,
- a represents 0 or 1,
- b represents 0 or 1,
- c represents an integer from 0 to 3,
- D represents an alkyl group, a cycloalkyl group or an alkyl or cycloalkyl group containing one or a number of substituents, such as:
- alkoxy radical having 1 to 4 carbon atoms,
- halogen atom,
- hydrophilic group, such as:
- COOM, -SO₃M or -PO₃M, M representing an inorganic or organic cationic residue chosen from the proton, cations derived from alkali or alkaline-earth metals, ammonium cations -N(R)₄, in the formula of which the R symbols, which are identical or different, represent a hydrogen atom or an alkyl radical having from 1 to 4 carbon atoms, and other cations derived from metals, the arylcarboxylic acid, arylsulphonic acid or arylphosphonic acid salts of which are soluble in water,
- N(R)₃, in the formula of which the R symbols, which are identical or different, represent a hydrogen atom or an alkyl radical having from 1 to 4 carbon atoms,
- OH
- d represents an integer from 0 to 3,
- the sum (a+b+c+d) is equal to 3
or to the general formula (II): 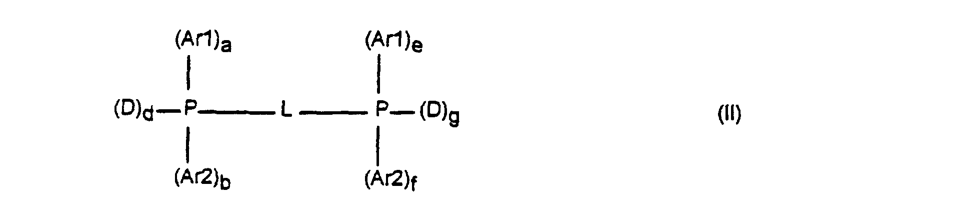
in which:
- Ar1, Ar2 and D have the meanings indicated above for the formula (I),
- a, b, e and f each represent 0 or 1,
- d and g each represent an integer from 0 to 2,
- the sum (a+b+d) is equal to 2,
- the sum (e+f+g) is equal to 2,
- L represents a single valency bond or a divalent hydrocarbon radical, such as an alkylene radical, a cycloalkylene radical, a phenylene radical, a diphenylene radical or a radical deriving from a heterocycle containing one or two oxygen, nitrogen or sulphur atoms in the ring, these various cyclic radicals being bonded directly to one of the phosphorus atoms or to both phosphorus atoms or being bonded to one of the phosphorus atoms or to both via a linear or branched alkylene radical having from 1 to 4 carbon atoms, it being possible for the ring or rings which are optionally part of the divalent radical L to contain one or a number of substituents, such as an alkyl group having 1 to 4 carbon atoms.

2. Process according to Claim 1, characterized in that the water-soluble phosphines are the phosphines of formula (I) or of formula (II) in which Ar1 and Ar2 are phenyl groups or phenyl groups containing one or two substituents as defined above, Ar3 is a phenyl group containing one or two substituents as defined above, D is an alkyl group having from 1 to 6 carbon atoms, a cycloalkyl group having 5 to 8 carbon atoms, an alkyl group having from 1 to 6 carbon atoms containing one or a number of substituents as defined above or a cycloalkyl group having 5 to 8 carbon atoms containing one or a number of substituents as defined above and L is a single valency bond, an alkylene radical having from 1 to 6 carbon atoms, a monocyclic or bicyclic cycloalkylene radical having from 4 to 12 carbon atoms, a phenylene radical, a diphenylene radical or a radical deriving from a heterocycle containing one or two oxygen, nitrogen or sulphur atoms in the ring, these various cyclic radicals being bonded directly to one of the phosphorus atoms or to both phosphorus atoms or being bonded to one of the phosphorus atoms or to both via a linear or branched alkylene radical having from 1 to 4 carbon atoms, it being possible for the ring or rings which are optionally part of the divalent radical L to contain one or a number of substituents, such as an alkyl group having 1 to 4 carbon atoms.

3. Process according to either of Claims 1 and 2, characterized in that the water-soluble phosphines are the phosphines of formula (I) or of formula (II) in which:
- the substituent or substituents of Ar1 and Ar2, which are identical or different, represent groups such as:
- alkyl or alkoxy radical having 1 to 2 carbon atoms,
- chlorine atom,
- hydrophilic group, such as:
- COOM, -SO₃M or -PO₃M, M representing an inorganic or organic cationic residue chosen from the proton, cations derived from sodium, potassium, calcium or barium, ammonium, tetramethylammonium, tetraethylammonium, tetrapropylammonium or tetrabutylammonium cations and cations derived from zinc, lead or tin,
- N(R)₃, in the formula of which the R symbols, which are identical or different, represent a hydrogen atom or an alkyl radical having from 1 to 4 carbon atoms,
- OH
- the substituent or substituents of Ar3, which are identical or different, represent groups such as:
- alkyl or alkoxy radical having 1 to 2 carbon atoms,
- chlorine atom,
- hydrophilic group, such as:
- COOM or -PO₃M, M representing an inorganic or organic cationic residue chosen from the proton, cations derived from sodium, potassium, calcium or barium, ammonium, tetramethylammonium, tetraethylammonium, tetrapropylammonium or tetrabutylammonium cations and cations derived from zinc, lead or tin,
overall at least two of the said substituents of Ar1, Ar2, Ar3 and D, for the phosphines of formula (I), and of Ar1, Ar2 and D, for the phosphines of formula (II), being a hydrophilic group as defined above.

4. Process according to one of Claims 1 to 3, characterized in that the transition metal compounds are chosen from compounds of nickel, of palladium and of iron which are soluble in water or capable of dissolving under the reaction conditions.

5. Process according to one of Claims 1 to 4, characterized in that the preferred transition metal compounds are those of nickel and are chosen from:
- compounds in which the nickel is in the zero oxidation state, such as potassium tetracyanonickelate K₄[(Ni(CN)₄], bis(acrylonitrile)nickel(0),
bis (1,5-cyclooctadiene)₂nickel and derivatives containing ligands from group Va, such as tetrakis(triphenylphosphine)nickel(0);
- nickel compounds such as carboxylates, carbonate, bicarbonate, borate, bromide, chloride, citrate, thiocyanate, cyanide, formate, hydroxide, hydrophosphite, phosphite, phosphate and derivatives, iodide, nitrate, sulphate, sulphite, arylsulphonates and alkylsulphonates.

6. Process according to one of Claims 1 to 5, characterized in that the organic compounds containing at least one ethylenic double bond are chosen from diolefins, such as butadiene, isoprene, 1,5-hexadiene or 1,5-cyclooctadiene, aliphatic nitriles containing ethylenic unsaturation, particularly linear pentenenitriles, such as 3-pentenenitrile or 4-pentenenitrile, monoolefins, such as styrene, methylstyrene, vinylnaphthalene, cyclohexene or methylcyclohexene, and mixtures of a number of these compounds.

7. Process according to one of Claims 1 to 6, characterized in that the amount of nickel compound or compound of another transition metal used is chosen so that there is, per litre of reaction solution, between 10⁻⁴ and 1, and preferably between 0.005 and 0.5, mol of nickel or of the other transition metal employed and in that the amount of water-soluble phosphine of formula (I) or (II) used is chosen so that the number of moles of this compound with respect to 1 mol of transition metal is from 0.5 to 2000 and preferably from 2 to 300.

8. Process according to one of Claims 1 to 7, characterized in that the hydrocyanation reaction is carried out at a temperature from 10°C to 200°C and preferably from 30°C to 120°C.

9. Process according to one of Claims 1 to 8 for the hydrocyanation of compounds containing ethylenic unsaturation by reaction with hydrogen cyanide, characterized in that the reaction is carried out in the presence of an aqueous solution of a catalyst comprising at least one transition metal compound, at least one water-soluble phosphine of formula (I) or (II) and a cocatalyst comprising at least one Lewis acid.

10. Process according to Claim 9, characterized in that the compounds containing ethylenic unsaturation are chosen from aliphatic nitriles containing ethylenic unsaturation, preferably linear pentenenitriles such as 3-pentenenitrile, 4-pentenenitrile and their mixtures.

11. Process according to Claim 10, characterized in that the linear pentenenitriles contain amounts, generally minor, of other compounds, such as 2-methyl-3-butenenitrile, 2-methyl-2-butenenitrile, 2-pentenenitrile, valeronitrile, adiponitrile, 2-methylglutaronitrile, 2-ethylsuccinonitrile or butadiene.

12. Process according to one of Claims 9 to 11, characterized in that the Lewis acid employed as cocatalyst is chosen from compounds of the elements from groups Ib, IIb, IIIa, IIIb, IVa, IVb, Va, Vb, VIb, VIIb and VIII of the Periodic Classification of the Elements, insofar as the said compounds are at least partially soluble and stable in water.

13. Process according to one of Claims 9 to 12, characterized in that the Lewis acid is chosen from salts, in particular halides, sulphates, carboxylates and phosphates.

14. Process according to one of Claims 9 to 13, characterized in that the Lewis acid is chosen from zinc chloride, zinc bromide, zinc iodide, manganese chloride, manganese bromide, cadmium chloride, cadmium bromide, stannous chloride, stannous bromide, stannous sulphate, stannous tartrate, chlorides or bromides of rare-earth metal elements, such as lanthanum, cerium, praseodymium, neodymium, samarium, europium, gadolinium, terbium, dysprosium, holmium, erbium, thulium, ytterbium and lutetium, cobalt chloride, ferrous chloride, yttrium chloride and their mixtures.

15. Process according to one of Claims 9 to 14, characterized in that the Lewis acid employed represents from 0.01 to 50 mol per mole of transition metal compound, more particularly of nickel compound, and preferably from 1 to 10 mol per mole.

16. Process according to one of Claims 1 to 15, characterized in that 2-methyl-3-butenenitrile present in the reaction mixture originating from the hydrocyanation of butadiene is isomerized to pentenenitriles in the absence of hydrogen cyanide, the isomerization being carried out in the presence of a catalyst containing at least one water-soluble phosphine of formula (I) or (II) and at least one transition metal compound.

17. Process according to Claim 16, characterized in that the 2-methyl-3-butenenitrile subjected to the isomerization is employed alone or as a mixture with 2-methyl-2-butenenitrile, 4-pentenenitrile, 3-pentenenitrile, 2-pentenenitrile, butadiene, adiponitrile, 2-methylglutaroronitrile, 2-ethylsuccinonitrile or valeronitrile.

18. Process according to either of Claims 16 and 17, characterized in that the isomerization reaction is carried out at a temperature from 10°C to 200°C and preferably from 60°C to 120°C.

19. Process according to one of Claims 16 to 18, characterized in that the isomerization of 2-methyl-3-butenenitrile to pentenenitriles is carried out in the presence of an aqueous solution of a catalyst comprising at least one transition metal compound, at least one water-soluble phosphine of formula (I) or (II) and a cocatalyst comprising at least one Lewis acid.
